# EUROPÄISCHE PATENTANMELDUNG

(11) **EP 0 983 984 A2**
(43) Veröffentlichungstag der Anmeldung: **08.03.2000**
(21) Anmeldenummer: 99116581.2
(22) Anmeldetag: 25.08.1999
(51) Int. Cl.: C07C 29/90, C07C 29/132, C07C 31/24

(54) **Verfahren zur reduzierenden Spaltung von linearen und cyclischen Acetalen, insbesondere Formalen**

(30) Priorität: 04.09.1998 DE 19840276
(71) Anmelder: Degussa-Hüls Aktiengesellschaft, 60287 Frankfurt am Main (DE)
(72) Erfinder: Morawietz, Marcus, Dr., 63457 Hanau (DE); Haas, Thomas, Dr., 60316 Frankfurt (DE); Burkhardt, Olaf, Dr., 63577 Alzenau (DE); Vanheertum, Rudolf, Dr., 63796 Kahl (DE)

(57) **Zusammenfassung**

Die reduzierende Spaltung von linearen und cyclischen Acetalen, insbesondere Formalen, in einem wäßrigen, ein Formiat enthaltenden Medium gelingt durch Hydrierung mit Wasserstoff in Gegenwart eines heterogenen Hydrierkatalysators bei einem pH-Wert unter 7 bei einer Temperatur von über 200 °C. Die katalysatorvergiftende Wirkung des Formiats wird bei über 200 bis 300 °C, insbesondere über 200 bis 280 °C, überwunden, so daß bei einer Suspensions-Fahrweise das Gewichtsverhältnis hydrierwirksames Metall zu Acetal viel niedriger als im Stand der Technik, nämlich kleiner 0,1 , sein kann.

## Beschreibung

Die Erfindung betrifft ein Verfahren zur reduzierenden Spaltung von linearen und cyclischen Acetalen, insbesondere Formalen. Die Erfindung richtet sich insbesondere auf die hydrogenolytische Spaltung von cyclischen Formalen in wäßrigem Medium in Gegenwart eines Formiats, insbesondere eines Ammonium-, Alkali- oder Erdalkaliformiats, wobei 1,3-Diole und Methanol gebildet werden.

Auch wenn die Hydrolyse von Formalen und anderen Acetalen zu den grundlegenden Reaktionen der organischen Chemie gehört, können sich bei einseitiger Gleichgewichtslage, wie sie beispielsweise bei cyclischen Acetalen mit einer 1,3-Dioxostruktur vorliegt, Probleme durch eine ungenügende Selektivität und Nebenreaktionen, darunter thermische Zersetzung, ergeben.

Lineare und cyclische Acetale, insbesondere Formale, entstehen als unerwünschte Nebenprodukte bei der großtechnischen Herstellung von mehrwertigen Alkoholen, wie Pentaerythritol, Trimethylolpropan und -ethan, Neopentylglykol und Oligomeren dieser Alkohole, welche eine Aldoladdition und eine Cannizzaroreaktion umfaßt. Bei der Cannizzaroreaktion, die in Gegenwart eines Alkali-, Erdalkali- oder Ammoniumhydroxids durchgeführt wird, entsteht das entsprechende Formiat. Letzteres ist in den unterschiedlichen Aufarbeitungsstufen der Polyole in unterschiedlicher Menge enthalten.

In der WO 97/01523 wird ein Verfahren zur hydrierenden Spaltung von cyclischen Formalen, wie sie bei der Aldoladdition mit nachfolgender Cannizzaroreaktion zur Herstellung mehrwertiger Alkohole, wie Pentaerythritol und Trimethylolpropan, als Nebenprodukte entstehen, zu den zugrundeliegenden Diolen und Methanol beschrieben. Die Spaltung erfolgt in wäßriger Phase bei pH 1 bis 6 in Gegenwart eines heterogenen Metallkatalysators bei 100 bis 200 °C in einer Wasserstoffatmosphäre. Ausweislich des Beispiels 1 dieses Dokuments wird ein zuvor angesäuertes wäßriges Reaktionsgemisch aus der Herstellung von Di-trimethylolpropan (Di-TMP), das außer den zu spaltenden cyclischen Formalen Di- und Tri-TMP und nicht identifizierten Verbindungen auch Natriumformiat enthält, in Gegenwart von Ru auf Aktivkohle bei 67 bar H₂ und 130 °C hydrogenolysiert.

Wie von den Erfindern der vorliegenden Anmeldung bei der Nacharbeitung der WO 97/01523 festgestellt wurde, ist diese Hydrogenolyse unter den genannten Druck- und Temperaturbedingungen nur erfolgreich durchführbar, wenn ein sehr hohes Gewichtsverhältnis katalytisch wirksames Metall zu cyclischem Formal - in dem genannten Beispiel 0,91 - zugrundegelegt wird. Offensichtlich wirkt das im Reaktionsgemisch enthaltende Formiat als Katalysatorgift. Eine derart hohe relative Katalysatoreinsatzmenge macht aber das Verfahren unwirtschaftlich, so daß die Aufgabe der Erfindung darin lag, das vorgenannte Verfahren zu verbessern.

Im fraglichen Verfahren sollte die Acetalspaltung möglichst quantitativ verlaufen, gleichzeitig sollte aber die Bildung ungesättigter und farbgebender Verbindungen vermieden werden.

Die Aufgabe wird gelöst durch ein Verfahren zur reduzierenden Spaltung von linearen und cyclischen Acetalen, insbesondere Formalen, in einem wäßrigen, ein Formiat enthaltenden Medium in Gegenwart eines heterogenen Hydrierkatalysators und Wasserstoff bei einem pH-Wert unter 7, einem Druck von 0,1 bis 30 MPa und einer Temperatur von über 100 °C, das dadurch gekennzeichnet ist, daß man die Umsetzung bei einer Temperatur von über 200 bis 300 °C durchführt, wobei im Falle einer Suspensions-Fahrweise das Gewichtsverhältnis hydrierwirksames Metall zu Acetal kleiner 0,1 ist. Die Unteransprüche richten sich auf bevorzugte Ausführungsformen.

Es wurde gefunden, daß die katalysatorvergiftende Wirkung der Formiate überraschenderweise allein durch Temperaturerhöhung behoben werden kann. Auf diese Weise ist es möglich, mit einem gegenüber dem Stand der Technik wesentlich niedrigeren Katalysatoreinsatz die hydrogenolytische Spaltung linearer und cyclischer Acetale mit hoher Selektivität des dem Acetal zugrundeliegenden 1,3-Diols durchzuführen. Hierbei ist es möglich, in Anwesenheit eines Fomiats nicht nur die Acetale per se zu spalten, sondern auch lineare und cyclische Formale, wie sie in Reaktionsgemischen aus der kombinierten Aldoladdition und Cannizzaroreaktion als Nebenprodukte enthalten sein können.

Die Untersuchungen zeigen, daß erst bei oder oberhalb einer Temperatur von 160 °C der negative Einfluß des Formiats auf den Hydrierkatalysator nahezu sprunghaft nachläßt und die Spaltung auch cyclischer Formale zum mehrwertigen Alkohol und Methanol ausreichend rasch und vollständig verläuft. Im allgemeinen liegt die Temperatur bei über 200 bis 300 °C und besonders bevorzugt erfolgt die Umsetzung bei über 200 bis 280 °C.

Überraschenderweise ist es auch möglich, eine ein Acetal enthaltende, bei Raumtemperatur sehr schwach saure (pH >6) oder auch neutrale wäßrige Lösung der erfindungsgemäßen Spaltung zuzuführen. Es muß also nicht zwingend ein saurer pH-Wert eingestellt werden, wie dies im vorbekannten Verfahren nötig war. Ein pH-Wert der Lösung von 7 (bei 20 °C) führt bei der angewandten Reaktionstemperatur zu einem Wert von kleiner 7. Die Anwesenheit eines sauren Katalysators im erfindungsgemäßen Verfahren wirkt sich allerdings nicht nachteilig, sondern etwas beschleunigend aus. Bei den Säuren kann es sich um protische anorganische oder organische Säuren, oder um saure Feststoffkatalysatoren handeln, deren Hₒ-Wert der Hammet'schen Säurefunktion kleiner +2, insbesondere kleiner -3, ist (Tanabe et al. in 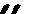 Surface science and catalysis" Vol. 51 (1989), 5). Mineralsäuren werden weniger bevorzugt, weil diese nach der Umsetzung neutralisiert und die Salze aus dem Reaktionsgemisch abgetrennt und entsorgt werden müssen. Bevorzugt werden im Falle eines Säureeinsatzes niedere Carbonsäuren, insbesondere Ameisensäure. Geeignet ist ein pH-Wert von zum Beispiel 1 bis 6. Beim Einsatz saurer Feststoffkatalysatoren, etwa solchen aus der Reihe natürlicher und synthetischer silikatischer Stoffe, wie Mordenit, Montmorillonit und saure Zeolithe, und Oxiden, Mischoxiden, Kationenaustauschern richtet sich die Einsatzmenge nach der Aktivität des Katalysators bei der gewählten Reaktionstemperatur. Saure Feststoffkatalysatoren können in Suspensionsform oder in Form eines Festbetts eingesetzt werden.

Das erfindungsgemäße Verfahren wird in Gegenwart eines üblichen Hydrierkatalysators bei einem Druck im Bereich von 0,1 bis 30 MPa durchgeführt. Zweckmäßigerweise liegt der Wasserstoffpartialdruck im Bereich von 0,5 bis 15 MPa, vorzugsweise 1 bis 5 MPa und insbesondere 1 bis 3 MPa. Heterogene Hydrierkatalysatoren werden bevorzugt, weil hiermit eine leichte Abtrennung des Katalysators vom Reaktionsgemisch möglich ist - Abtrennung eines Suspensionskatalysators durch Filtration beziehungsweise Anordnung des Katalysators als Festbett, etwa im Falle einer Rieselbett- oder Blasensäulefahrweise.

Übliche Hydrierkatalysatoren enthalten als wirksame Komponente ein Edelmetall aus der Reihe Ru, Rh, Pd und Pt, oder ein Übergangsmetall aus der Reihe Cu, Cr, Co, Ni, Fe, darunter insbesondere Raney-Katalysatoren und Chromit-Katalysatoren; einsetzbar, wenngleich wegen begrenzter Standzeit nicht bevorzugt, sind auch Bimetall-Katalysatoren aus einem Übergangsmetall und Edelmetall. Die Verwendung eines ein oder mehrere Übergangsmetalle enthaltenden Hydrierkatalysators ist nur dann zweckmäßig, wenn der Katalysator unter den Reaktionsbedingungen eine ausreichende Säurestabilität aufweist.

Bevorzugte Hydrierkatalysatoren für das erfindungsgemäße Verfahren sind Edelmetallkatalysatoren in metallischer Form, wie sogenannte Mohre von Ru, Rh und insbesondere Pd und Pt, oder in an einen Träger gebundener Form. Geeignete Trägermaterialien für Ru, Rh, Pd und Pt sind Aktivkohle, Aluminiumoxid, SiO₂, TiO₂ und andere Metalloxide sowie Silikate. Die Edelmetallmenge trägergebundener Edelmetallkatalysatoren liegt meist im Bereich von 0,0001 bis 10 Gew.-%, insbesondere im Bereich von 0,01 bis 5 Gew.-%. Die optimale Einsatzmenge an Edelmetallkatalysatoren, welche von der Aktivität des Katalysators, der Reaktionstemperatur und dem H₂-Druck abhängt, wird der Fachmann durch orientierende Versuche ermitteln. Es ist auch möglich, einen sowohl saure als auch hydrierwirksame Funktionen enthaltenden Katalysator, beispielsweise einen teilweise mit einem Edelmetall beladenen Zeolithen, einzusetzen. Ein Vorteil des erfindungsgemäßen Verfahrens besteht darin, daß bei Verwendung eines Suspensions-Hydrierkatalysators das Gewichtsverhältnis des hydrierwirksamen Metalls zum zu spaltenden Acetal kleiner 0,1 und vorzugsweise kleiner 0,01 ist. Bei Verwendung eines Festbett-Hydrierkatalysators kann bei Einhaltung der erfindungsgemäßen Temperatur mit technisch üblichen und eine gute Raum-Zeit-Ausbeute erlaubenden LHSV-Werten (liquid hourly space velocity) von beispielsweise gleich/größer 1 h⁻¹ gearbeitet werden.

Dem erfindungsgemäßen Verfahren zur Acetalspaltung sind Acetale von ein- oder mehrwertigen Alkoholen, insbesondere mehrwertigen primären Alkoholen oder solche Acetale enthaltende Gemische zugänglich. Die Acetale basieren vorzugsweise auf niederen aliphatischen oder aromatischen Aldehyden, besonders auf Formaldehyd; Acetale von Formaldehyd (= Formale) liegen oft in linearer oder cyclischer Form nebeneinander vor. Wesentlich ist, daß das Acetal beziehungsweise Formal unter den Reaktionsbedingungen eine ausreichende Löslichkeit in der wäßrigen, salzhaltigen Lösung aufweist. Die Acetale und Formale können weitere funktionelle Gruppen enthalten, soweit diese unter den erfindungsgemäßen Hydrierbedingungen nicht hydriert oder hydrolysiert werden.

Besonders bevorzugt lassen sich erfindungsgemäß aliphatische Acetale, insbesondere Formale aus der Reihe der 1,3-Dioxane und 1,3-Dioxolane, die auf Diolen, Triolen, Tetrolen und Polyolen und Polyolethern basieren, hydrogenolytisch spalten. Beispiele für Diole sind Ethylenglykol, Propylenglykol, Propan-1,3-diol, Butan-1,4-diol, Hexan-1,6-diol, Neopentylglykol; Beispiele für Triole sind Glycerin, Trimethylolethan (TME), Trimethlolpropan (TMP), Hexan-1,2,6-triol; ein Beispiel für Tetrole ist Pentaerythritol und Beispiele für Polyole sind Di- und Tri-TMP sowie Di- und Tripentaerythritol.

Die erfindungsgemäße Acetal- bzw. Formalspaltung erfolgt in einem wäßrigen Medium, das ein Formiat, insbesondere ein Alkali-, Ammonium- oder Erdalkaliformiat, enthält. Bei Bedarf kann die zu spaltende Lösung außerdem ein- oder mehrwertige Alkohole und/oder zusätzlich andere säure- und hydrierstabile Lösungsmittel, wie ein aprotisches dipolares Lösungsmittel, enthalten. Bevorzugt wird aber der Einsatz einer rein wäßrigen oder wäßrig-alkoholischen Lösung. Zweckmäßigerweise werden die Acetale und das Wasser im Gewichtsverhältnis im Bereich von 10 zu 1 bis 1 zu 100, vorzugsweise im Bereich von 2 zu 1 bis 1 zu 20 eingesetzt. Besonders bevorzugt werden Formale und ein Formiat enthaltende Reaktionsgemische, wie sie bei einer Aldoladdition mit nachfolgender Cannizzaro-Reaktion oder im Rahmen deren Aufarbeitung anfallen, eingesetzt.

Das Verfahren kann diskontinuierlich oder kontinuierlich betrieben werden. Bei kontinuierlicher Fahrweise und Einsatz eines Suspensions-Hydrierkatalysators kann die zu behandelnde ein Acetal und Formiat enthaltende Lösung kontinuierlich einem Umlaufreaktor, in welchem sich der Suspensionskatalysator befindet, zugeführt und ein entsprechender Teil über ein Filter ausgeschleust werden. Besonders bevorzugt wird das Verfahren unter Einsatz eines Festbett-Hydrierkatalysators kontinuierlich betrieben. Die Aufarbeitung des Reaktionsgemischs nach beendeter Umsetzung - die optimale Reaktionszeit wird der Fachmann durch orientierende Vorversuche ermitteln - erfolgt in dem Fachmann bekannter Weise unter Einsatz üblicher Schritte, wie Filtration, Kristallisation, Destillation. Die Analyse der Zusammensetzung des Reaktionsgemischs oder einzelner Fraktionen erfolgt über GC- oder HPLC-Chromatographie. Polyole und Polyolether werden vorzugsweise vor der Analytik mit Hexamethyldisilazan / DMF silyliert und anschließend mit GC-Chromatographie analysiert.

Vorteile des erfindungsgemäßen Verfahrens sind die hohe Selektivität bei der Bildung der Alkohole aus den Acetalen und der wesentlich niedrigere Katalysatoreinsatz beziehungsweise erhöhte Raum-Zeit-Ausbeute. Das Verfahren läßt sich einfach durchführen und die Verwendung eines sauren Katalysators ist optional.

Anhand der nachfolgenden Beispiele und Vergleichsbeispiele wird die Erfindung im Vergleich zum Stand der Technik weiter verdeutlicht.

### Vergleichsbeispiel 1 (VB 1)

### Versuch der Umsetzung von Pentaerythritolmonoformal zu Pentaerythritol in Gegenwart von Natriumformiat analog dem in der WO 97/10523 beschriebenen Verfahren

Es wurden 100 g Pentaerythritmonoformal, 23 g Natriumformiat (2 Gew.-% der Reaktionslösung), 1000 g 0,1 % wäßrige H₃PO₄-Lösung und 10,1 g Hydrierkatalysator - 3 % Ru auf Aktivkohle - in einen 2L-Autoklav gegeben. Bei Raumtemperatur wurden 4 MPa Wasserstoff aufgepreßt und anschließend wurde das Reaktionsgemisch auf 150 °C erwärmt, dabei steigt der Druck auf ca. 6 MPa. Bei dieser Temperatur wurde das Gemisch 180 min. gerührt. Nach dem Abkühlen wurde der Autoklav entleert, der Katalysator abfiltriert und eine repräsentative Probe entnommen. Das Wasser wurde am Rotationsverdampfer vollständig entfernt, es wurde eine farblose Flüssigkeit erhalten und der Rückstand mittels GC quantitativ analysiert. Die Formalspaltung war völlig unzureichend.

Die Analysendaten sind der Tabelle zu entnehmen.

### Vergleichsbeispiel 2 (VB 2)

### Versuch der Umsetzung von Pentaerythritolmonoformal zu Pentaerythritol in Gegenwart von Calciumformiat analog dem in der WO 97/10523 beschriebenen Verfahren

Die Reaktion wurde analog dem Vergleichsbeispiel 1 durchgeführt, nur mit dem Unterschied, daß anstelle von Natriumformiat 23 g Calciumformiat eingesetzt wurden. Auch hier war der Formalumsatz völlig unzureichend.

Die Analysendaten sind der Tabelle zu entnehmen.

### Beispiel 1 (B 1)

### Erfindungsgemäße Umsetzung von Pentaerythritolmonoformal zu Pentaerythritol in Gegenwart von Calciumformiat

Es wurden 25 g Pentaerythritolmonoformal, 500 g Wasser und 25 g Calciumformiat zusammengegeben, das Gemisch mit wenig Ameisensäure auf pH 5 eingestellt, das Gemisch nach Zugabe von 3 g Hydrierkatalysator - 5 % Ru auf Aktivkohle - in einen 1L-Autoklav gegeben. Bei Raumtemperatur wurden 2 MPa Wasserstoff aufgepreßt und anschließend wurde das Reaktionsgemisch auf 280 °C erwärmt (Druckanstieg auf ca. 10 MPa) . Bei dieser Temperatur wurde das Gemisch 60 min. gerührt. Nach dem Abkühlen wurde der Autoklav entleert, der Katalysator abfiltriert und eine repräsentative Probe entnommen. Das Wasser wurde am Rotationsverdampfer vollständig entfernt, es wurde ein farbloser Feststoff erhalten. Der Rückstand wurde mittels GC quantitativ analysiert. Die Umsetzung, also Spaltung des Formals zu Pentaerythritol, war nahezu quantitativ.

Die Analysendaten sind der Tabelle zu entnehmen.

### Beispiel 2 (B 2)

### Umsetzung einer Pentaerythritolformale, Pentaerythrit und höhere Polyole enthaltenden Lösung aus einem technischen Prozeß zur Herstellung von Pentaerythrit aus Acetalaldehyd, Formaldehyd und Calciumhydroxid

Es wurden 500 g einer Lösung mit folgender Zusammensetzung:
- 4 Gew.-%: lineare und cyclische Pentaerythritformale
- 18 Gew.-%: Pentaerythrit
- 3 Gew.-%: Di- / Tripentaerythrit
- 8 Gew.-%: Calciumformiat
- 67 Gew.-%: Wasser
und 3 g Hydrierkatalysator - 5 % Ru auf Aktivkohle - mit wenig Ameisensäure auf pH 5-6 eingestellt und in einen 1L-Autoklav gegeben. Bei Raumtemperatur wurden 2 MPa Wasserstoff aufgepreßt; anschließend wurde das Reaktionsgemisch auf 250 °C erwärmt (Druckanstieg auf ca. 8,5 MPa). Bei dieser Temperatur wurde das Gemisch 120 min. gerührt. Nach dem Abkühlen wurde der Autoklav bei 40 °C entleert, der Katalysator abfiltriert und eine repräsentative Probe entnommen. Das Wasser wurde am Rotationsverdampfer vollständig entfernt, es wurde ein farbloser Feststoff erhalten; der Rückstand wurde mittels GC quantitativ analysiert. Die Formale wurden fest quantitativ gespalten und Pentaerythrit entstand mit hoher Selektivität.

Die Analysendaten sind der Tabelle zu entnehmen.

### Beispiel 3 (B 3)

Beispiel 2 wurde wiederholt, wobei jedoch die Umsetzung bei 280 °C über 60 min. anstelle 250 °C / 120 min. erfolgte. Auch hier wurde ein vergleichbar gutes Ergebnis erzielt.

Die Analysendaten sind der Tabelle zu entnehmen.

### Vergleichsbeispiel 3 (VB 3)

### Umsetzung eines Gemischs aus Pentaerythrit, Pentaerythritolformalen und höheren Polyolen zu einer Mischung aus Pentaerythritol und höheren Polyolen in Gegenwart von wenig Calciumformiat

Es wurden 500 g einer Lösung mit folgender Zusammensetzung:
- 4 Gew.-%: lineare und cyclische Pentaerythritformale
- 29 Gew.-%: Pentaerythrit
- 4 Gew.-%: Di- / Tripentaerythrit
- 0,5 Gew.-%: Calciumformiat
- 62,5 Gew.-%: Wasser
und 3 g Hydrierkatalysator - 5 % Ru auf Aktivkohle - mit wenig Ameisensäure auf pH 5-6 eingestellt und in einen 1L-Autoklav gegeben. Bei Raumtemperatur wurden 2 MPa Wasserstoff aufgepreßt und anschließend das Reaktionsgemisch auf 140 °C erwärmt. Bei dieser Temperatur wurde das Gemisch 180 min. gerührt. Nach dem Abkühlen wurde der Autoklav bei 40 °C entleert, der Katalysator abfiltriert und eine repräsentative Probe entnommen. Das Wasser wurde am Rotationsverdampfer vollständig entfernt, es wurde ein farbloser Feststoff erhalten und der Rückstand wurde mittels GC quantitativ analysiert. Die Umsetzung erfolgte sehr unvollständig.

Die Analysendaten sind der Tabelle zu entnehmen.

### Vergleichsbeispiel 4 (VB 4)

500 g Lösung der Zusammensetzung des Vergleichsbeispiels 3 wurde mit der in VB 3 angegebenen Katalysatormenge bei 150 °C über 180 min. (anstelle 140 °C / 180 min.) hydrogenolytisch behandelt. Es konnte nur ein geringer Formalumsatz nachgewiesen werden.

Die Analysendaten sind der Tabelle zu entnehmen.

### Beispiel 4 (B 4)

500 g Lösung der Zusammensetzung des Vergleichsbeispiels 3 wurde mit der in VB 3 angegebenen Katalysatormenge erfindungsgemäß bei 180 °C über 120 min. hydrogenolytisch behandelt. Erhalten wurden ein sehr hoher Formalumsatz sowie eine sehr hohe Selektivität.

Die Analysendaten sind der Tabelle zu entnehmen.

Der prinzipielle Unterschied und Effekt des erfindungsgemäßen Verfahrens (B 4) im Vergleich zum vorbekannten Verfahren (VB 3 und VB 4) wird besonders deutlich, wenn die aus dem Umsatz und der Selektivität (siehe Tabelle) ermittelbare Ausbeute an Pentaerythritol betrachtet wird: VB 3 (140 °C) ca. 1%, VB 4 (150 °C) ca. 10 % und B 4 ca. 86 %.

### Beispiel 5 (B 5)

500 g Lösung der Zusammensetzung des Vergleichsbeispiels 3 wurde mit der in VB 3 angegebenen Katalysatormenge erfindungsgemäß bei 200 °C über 120 min. hydrogenolytisch behandelt, allerdings ohne vorher die Lösung mit Ameisensäure auf pH = 5-6 einzustellen. Erhalten wurde ein sehr hoher Formalumsatz sowie eine sehr hohe Selektivität.

Die Analysendaten sind der Tabelle zu entnehmen.

**Tabelle**

| Nr. | Umsatz (% bez. einges. Formale) | Selektivität Pentaerythritol (% bez. umges. Formale) |
|---|---|---|
| VB 1 | < 10 | < 11 |
| VB 2 | 36 | 11 |
| B 1 | > 99 | 98 |
| B 2 | 95 | 91 |
| B 3 | 98 | 90 |
| VB 3 | < 5 | 25 |
| VB 4 | 35 | 31 |
| B 4 | 93 | 94 |
| B 5 | 90 | 89 |

## Patentansprüche

1. Verfahren zur reduzierenden Spaltung von linearen und cyclischen Acetalen, insbesondere Formalen, in einem wäßrigen, ein Formiat enthaltenden Medium in Gegenwart eines heterogenen Hydrierkatalysators und Wasserstoff bei einem pH-Wert unter 7, einem Druck von 0,1 bis 30 MPa und einer Temperatur von über 100 °C,
dadurch gekennzeichnet,
daß man die Umsetzung bei einer Temperatur von über 200 bis 300 °C durchführt, wobei im Falle einer Suspensions-Fahrweise das Gewichtsverhältnis hydrierwirksames Metall zu Acetal kleiner 0,1 ist.

2. Verfahren nach Anspruch 1,
dadurch gekennzeichnet,
daß man die Umsetzung bei einer Temperatur von über 200 bis 280 °C durchführt.

3. Verfahren nach Anspruch 1 oder 2,
dadurch gekennzeichnet,
daß man ein cyclische und/oder lineare Formale als Nebenprodukte und Natrium-, Calcium- oder Ammoniumformiat als Koppelprodukt enthaltendes Reaktionsgemisch aus einer Aldoladdition und Cannizzaroreaktion der reduzierenden Spaltung zuführt.

4. Verfahren nach einem oder mehreren der Ansprüche 1 bis 3,
dadurch gekennzeichnet,
daß man als Katalysator einen Trägerkatalysator auf der Basis einer Aktivkohle oder eines oxidischen Trägers mit einem oder mehrere hydrierwirksamen Metall(en) aus der Reihe Ru, Rh, Pd, Pt, Cu, Cr, Fe, Co und Ni benutzt.

5. Verfahren nach Anspruch 4,
dadurch gekennzeichnet,
daß man als Katalysator Ru und/oder Pd auf Aktivkohle verwendet.

6. Verfahren nach einem oder mehreren der Ansprüche 1 bis 5,
dadurch gekennzeichnet,
daß man die der hydrierenden Spaltung zuzuführende wäßrige Lösung auf einen pH-Wert von 4 bis 7, insbesondere größer 6 bis 7, einstellt.

7. Verfahren nach einem oder mehreren der Ansprüche 1 bis 6,
dadurch gekennzeichnet,
daß man die hydrierende Spaltung unter Einsatz eines Festbettkatalysators in Rieselbett- oder Blasensäulefahrweise durchführt.

8. Verfahren nach einem oder mehreren der Ansprüche 1 bis 7,
dadurch gekennzeichnet,
daß man die Spaltung bei einem H₂-Partialdruck von 1 bis 5 MPa, insbesondere 1 bis 3 MPa, durchführt.
